# EUROPEAN PATENT SPECIFICATION

(45) Date of publication of patent specification: **20.12.95**

(51) Int. Cl.[6]: **A61K 39/21**, C12P 21/08, G01N 33/569

(21) Application number: **89401498.4**

(22) Date of filing: **31.05.89**

Divisional application 95108885.5 filed on 31/05/89.

(54) **Proteins and glycoproteins of the HIV-2 EHO retrovirus antiobodies directed against them - application for the diagnosis**

(43) Date of publication of application:
**05.12.90 Bulletin 90/49**

(45) Publication of the grant of the patent:
**20.12.95 Bulletin 95/51**

(84) Designated Contracting States:
**AT BE CH DE ES FR GB GR IT LI LU NL SE**

(56) References cited:
**EP-A- 0 253 701**
**EP-A- 0 283 327**
**WO-A-88/08449**

**COMTE RENDU DE L'ACADEMIE DES SCIENCES DE PARIS, vol. 302, Série III, no. 13, 1986, Académie des Sciences; F. CLAVEL et al., pp. 485-488&NUM;**

**PROCEEDINGS OF THE NATL. ACADEMY OF SCIENCES USA, vol. 85, August 1988; J.F. ZAGURY et al., pp. 5941-5945&NUM;**

**NATURE, vol. 326, 16 April 1987; M. GUYADER et al., pp. 662-669&NUM;**

(73) Proprietor: **INSTITUT PASTEUR**

25-28, rue du Docteur Roux
F-75724 Paris Cédex 15 (FR)

(72) Inventor: **Hovanessian, Ara**
**79 rue Ernest Savart**
**F-93100 Montreuil (FR)**
Inventor: **Rey, Marie-Anne**
**10 rue du Temple**
**F-75004 Paris (FR)**
Inventor: **Laurent, Anne**
**9 avenue Emile Deschanel**
**F-75007 Paris (FR)**
Inventor: **Krust, Bernard**
**7 rue de Madagascar**
**F-75012 Paris (FR)**
Inventor: **Guetard, Denise**
**48 rue Anselme Payen**
**F-75015 Paris (FR)**
Inventor: **Montagnier, Luc**
**21 rue de Malabry**
**F-92350 Plessis-Robinson (FR)**

(74) Representative: **Desaix, Anne et al**
**Ernest Gutmann - Yves Plasseraud S.A.**
**3, rue Chauveau-Lagarde**
**F-75008 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

Human immunodeficiency virus (HIV) is considered to be the etiologic agent of the acquired immunodeficiency syndrome (AIDS) (Montagnier et al. 1984). To date, two related but distinct types HIV-1 and HIV-2, have been identified (Barré Sinoussi et al 1983, Popovic et al, 1984, Ratner et al. 1985, Wain Hobson et al. 1985, Clavel et al 1986a, Brun-Vézinet et al, 1987).

HIV-2 has been described in european patent application n° 0239425 filed on January 22, 1987. At this time it was pointed out that HIV-2 is closely related to the simian immunodeficiency virus (SIV-mac), which causes an AIDS like disease in macaques. Alignments of the nucleotide sequences of HIV-1, HIV-2 and SIV reveal a considerable homology between HIV-2 and SIV-mac. These two viruses share about 75% overall nucleotide sequence homology, both of them are only distantly related to HIV-1 with about 40% overall homology see also (Guyader et al. 1987 ; Chakrabarti et al. 1977). In addition to the genes that encode structural proteins (the virion capsid and envelope proteins) and the enzymes required for proviral synthesis and integration common to all retroviruses, HIV-1, HIV-2 and SIV encode genes that regulate virus replication as well as genes that encode proteins to yet unknown function. The only notable difference in the genetic organizations of HIV-1, HIV-2 and SIV resides in the open reading frame referred to as vpx, which is absent in HIV-1 and vpu which is present in HIV-1 but not in HIV-2 and SIV (Cohen et al. 1988 ; Guyader et al. 1987). These viruses are both tropic and cytopathic for CD4 positive T lymphocytes (Klatzmann et al. 1984 ; Clavel et al. 1986a ; Dalgleish et al. 1984 ; Daniel et al. 1985). A gtreat number of studies have indicated that CD4 functions as the cellular receptor of HIV (Weiss, 1988).

In other studies, it has been reported that HIV-2 and SIV mac can be differentiated from HIV-1 by the processing pathway of their envelope glycoprotein precursors. HIV-2 and SIV envelope precursors form an homologous dimer (which is a glycoprotein gp300 for HIV-2 ROD) during their processing into the mature products, the extracellular and the transmembrane glycoproteins gp125 and gp 36 respectively (Rey et al. J. Virol. 63/647.658 1989a). Furthermore, transmembrane glycoproteins of HIV-2 and SIV exist as homodimers having a molecular weight of about 80 kDa for HIV-2 ROD. Under similar experimental conditions, dimerization of the envelope precursor and dimeric forms of the transmembrane glycoprotein are not observed in the case of HIV-1. Therefore we can consider that dimerization of the envelope glycoproteins is a specific property of HIV-2 and SIV envelope gene expression. Accordingly, this property could be used as a convenient marker to differentiate between different isolates of HIV and SIV.

The inventors working further on the human HIV-2 retroviruses have now demonstrated that there could be some differences among the various isolates of HIV-2. In accordance with that they have defined subtypes of the retrovirus HIV-2.

A first subgroup can be related to the isolate HIV-2 ROD deposited with the CNCM under n° I 532 on February 21, 1986 and with the ATCC under n° 87011002 on January 9, 1987.

A second subgroup can be related to the isolate HIV-2 EHO, deposited with the CNCM under n° I 643 on December 19, 1986.

These two isolates have been described in the european patent application 0239425 and have been characterised by their common properties.

The inventors have been more particularly interested in studying the properties of a specific isolate of HIV-2 : HIV 2 EHO. According to the definition already given for the HIV-2 retrovirus type, HIV-2 EHO produces a 14kDa protein related to the product of the vpx gene of HIV-2 ROD.

The inventors have now pointed out a surprising property of HIV-2 EHO, related to the structure of its envelope precursor and to its mature products, the transmembrane and extracellular glycoproteins and corresponding proteins.

The invention is therefore directed to new antigens constituted by proteins and glycoproteins characteristic of the HIV-2 EHO subtype of the HIV-2 retrovirus.

The invention also concerns compositions comprising the above antigens and antibodies recognizing these antigens.

Moreover the invention provides new means for the in vitro diagnosis of an infection by a HIV-2 EHO retrovirus or a related retrovirus, involving these antigens.

The invention concerns also a process for the preparation of the antigens and antibodies cited above.

Surprisingly, the inventors have given evidence that the envelope precursor and the mature products, that is the extracellular and transmembrane glycoproteins of HIV-2 EHO which distinguish from the corresponding antigens of HIV-2 ROD by a difference in their molecular weights and the absence of cross reactivity between the HIV-2 EHO envelope glycoproteins or precursors or glycoproteins having the same immunological properties and, determined particular antibodies directed against HIV-2 ROD envelope glycoproteins or precursors of these glycoproteins.

The invention accordingly concerns a purified antigen of the human HIV-2 EHO retrovirus characterised in that it is constituted by the envelope glycoprotein of HIV-2 EHO or a glycoprotein of a variant thereof or part of said antigen, or an antigen having the same immunological properties, which antigen is recognizable by human HIV-2 positive serum which is capable of recognizing the antigens of HIV-2 ROD, in an immunoprecipitation assay or in a western blot assay and it is not recognized by antibodies directed against the glycoprotein gp300 of HIV-2 ROD under the same conditions in an immunoprecipitation assay or in a western blot assay.

A typical human HIV-2 positive serum as mentioned hereabove, is a serum which is capable of recognizing the antigens of HIV-2 ROD.

The molecular weights of the various proteins and glycoproteins of the present invention could vary in a range of ±10% of the indicated molecular weight.

In a first aspect of the invention a purified antigen having the above characteristics is defined as a purified antigen of the HIV-2 EHO retrovirus or a glycoprotein having the same immunological properties and obtained from a variant of the HIV-2 EHO retrovirus , characterised in that it is encoded by the env gene and in that it has a molecular weight of the order of 120 kDa corresponding to the envelope precursor.

In a second aspect of the invention a purified antigen of the HIV-2 EHO retrovirus or a glycoprotein having the same immunological properties and obtained from a variant of the HIV-2 EHO retrovirus is characterised in that it is a glycoprotein gp270 corresponding to the dimeric form of the gp120 and in that it has a molecular weight of the order of 270 kDa.

This antigen represents the dimeric form of the precursor of the HIV-2 EHO glycoprotein gp120 which is firstly synthesized during the process of formation of the virus. This antigen seems to be necessary for the formation of the viral particles.

A third glycoprotein which is concerned by the invention is a purified antigen of the HIV-2 EHO retrovirus or a glycoprotein having the same immunological properties and obtained from a variant of the HIV-2 EHO retrovirus, characterised in that it corresponds to the mature product of the envelope precursor gp120 and in that it is the extracellular envelope glycoprotein gp100 having a molecular weight of the order of 100 kDa.

The characterisation by the inventors of the above glycoproteins related to the formation and maturation of the envelope glycoprotein gp100 of HIV-2 EHO, has enable them to conclude to a general difference of about 20 to 30kDalton (kDa) between the apparent molecular weights of the glycoproteins of HIV-2 EHO and the corresponding one of HIV-2 ROD.

They have further investigated to determine the origin of this difference. Since there are at least 30 potential N-linked glycosylation sites on the envelope precursor gp140 of HIV-2 ROD, it remained possible that the variation of the molecular weight between the glycoproteins related to the envelope glycoproteins of HIV-2 EHO on the one hand and of HIV-2 ROD on the other hand, be associated with a reduced number of oligosaccharide chains in the above glycoproteins of HIV-2 EHO.

Surprisingly the inventors have demonstrated that the variations of the molecular weights of the various glycoproteins involved in the formation of the envelope of HIV-2 EHO is due to differences in the polypeptide patterns of the glycoproteins, which reflect differences in the amino-acid composition between both envelope precursors.

Therefore the invention also concerns a purified antigen of the HIV-2 EHO retrovirus or an unglycosylated product of the glycoprotein having the same immunological properties and obtained from a variant of the HIV-2 EHO retrovirus characterised in that it is a protein p60 and it has the same aminoacids composition as the glycoprotein gp120 and in that it has no oligosaccharide chains.

In an other aspect of the invention any of the proteins derived from the above gp120, gp270, gp100 glycoproteins are concerned, providing that these proteins are free of the oligosaccharide chains present in the glycoproteins.

Such a protein could present particular epitopes which could usually not be accessible in the corresponding glycoprotein.

The above described proteins and glycoproteins can be included, alone or in any possible combination, in a composition for the in vitro diagnosis of an infection especially due to a human retrovirus of the HIV-2 EHO subtype.

In a first preferred embodiment, the composition defined hereabove is characterised in that it contains the precursor gp120 of the envelope glycoprotein, having a molecular weight of the order of 120 kDa.

In a second preferred embodiment the composition contains the extracellular envelope glycoprotein gp100, having a molecular weight of the order of 100 kDa, or it contains this gp100 in association with the above gp120.

In another embodiment according to the invention, the composition contains the dimeric glycoprotein gp270, having a molecular weight of the order of 270 kDa. This composition can further contains one of the above described glycoproteins or a combination thereof.

Other compositions which are in accordance with the definitions of the invention, are those containing proteins derived from the above glycoproteins and which are characterised by the absence of oligosaccharide chains.

One of the convenient proteins for the realisation of said compositions is the protein p60 above defined.

The invention also concerns a purified antigen of HIV-2 EHO in accordance with the above general definition, or a glycoprotein having the same immunological properties and obtained from a variant of HIV-2 EHO, characterised in that it is the transmembrane glycoprotein gp36, having a molecular weight, of the order of 36 kDa.

Another antigen according to the invention and which is related to the preceding one, or a glycoprotein having the same immunological properties, and obtained from a variant of HIV-2 EHO, is characterised in that it is a glycoprotein gp80 and it corresponds to the dimeric form of the gp36.

The invention also concerns an immunogenic composition comprising antigens of HIV-2 EHO as described in the preceding pages or antigens having the immunological properties of HIV-2 EHO antigens. Such composition could be used as vaccine. In this case they are prepared to allow the administration of a dose of 50 to 100μg per kilogram.

Compositions corresponding to the above definition comprise for example the glycoprotein gp100, which could be associated with others of the glycoproteins gp270, gp120, gp80 or gp36.

A preferred immunogenic composition according to the invention comprises further the corresponding antigens of HIV-2 ROD especially chosen among gp300, gp140, gp129, gp80 or gp36.

The invention also concerns polyclonal antibodies obtained from animals and monoclonal antibodies directed against the antigens defined hereabove.

WO-A-9 013 314, which is cited under Article 53(3) EPC, discloses on pages 24-25 the preparation of that monoclonal antibody referred to as "mabH18" on page 25 thereafter. According to this document the said antibody reacts specifically with the amino acid sequence of the transmembrane glycoprotein of HIV-2 ROD comprised between amino acid residues at positions 579 and 604.

The monoclonal antibodies according to the invention are, in a first embodiment, characterised in that they recognize a common epitope to the purified antigens gp120, gp270, gp100.

In a second embodiment these monoclonal antibodies are characterised in that they recognize specifically the glycoprotein gp100.

In a third preferred embodiment these monoclonal antibodies are characterised in that they recognize specifically the glycoprotein gp120.

In another preferred embodiment these monoclonal antibodies are characterised in that they recognize specifically the glycoprotein gp270.

More particularly the antibodies above described are such that they are directed against epitopes which are present on one or several antigens of the invention, these epitopes being not recognized by antibodies against the gp300 or one of the envelope related glycoproteins gp140 or gp125 of HIV-2 ROD.

Several processes for the production of antibodies can be employed according to the invention. For instance use can be made of ascitic cultures in animals, especially in rodents like mice or murine. Use can also be made of cellular cultures, either immobilysed or encapsulated or in suspension. The technique of the hybridomas obtained described by Kohler and Milstein (Nature 256 495-497) can be used. According to this, a process for preparing the hybridomas for the manufacture of the monoclonal antibodies of the invention comprises :

- starting from spleen cells of an animal, e.g. mouse or rat, previously immunized in vivo or from spleen cells of such animals previously immunized in vitro with an antigen of the invention.
- fusing said immunized cells with myeloma cells under hybridoma forming conditions and
- selecting those of the hybridomas which secrete the monoclonal antibodies which specifically recognize the above defined antigens.

The selection of the hybridomas can be realised by conventional techniques involving assays for the ability of hybridomas to produce antibodies against the determined antigens.

A process for producing the monoclonal antibodies directed against one or several antigens defined hereabove, comprises :

- culturing the selected hybridomas as indicated above in an appropriate culture medium and
- recovering the monoclonal antibodies excreted by said selected hybridomas, or alternatively
- implanting the selected hybridomas into the peritoneum of a mouse and, when ascites have been produced in the animal,

- recovering the monoclonal antibodies then formed from said ascites.

The antibodies can be recovered by various techniques such as radioimmuno-assay with the antigens to be recognized.

In another provision, the invention concerns a method for the preparation of a purified antigen having the above mentioned characteristics comprising the following steps :

- lysing the cells infected with a human retrovirus HIV-2 EHO or HIV-2 EHO viral pellets and separating the supernanant from the cellular extract,
- incubating the diluted extract with a serum of a patient infected with HIV-2 EHO or HIV-2 EHO viral pellets retrovirus or with polyclonal or monoclonal antibodies defined above, on an immunoaffinity column in conditions sufficient to allow the formation of a complex between said antibodies and antigens of the cellular extract,
- washing the immunoaffinity column to remove the molecules which are not retained on the support,
- resolving the eluted antigens.

The infected cells can be obtained in vitro after infection of cells for instance of CEM cells and after culture of these cells.

The resolution of the antigens can for instance by made by electrophoresis for example in polyacrylamide SDS gel.

The resolution of the proteins can for example be obtained by :

- elution of the proteins attached to the immunoaffinity column,
- purification of the products thus eluted on a chromatography column containing, bound to the support, monoclonal antibodies recognizing the antigen to be purified.

Preparation of these antigens can also be made with virus pellets.

In a preferred embodiment of the process for the preparation of the proteins, the lysis of the infected cells or virus pellets is realised in a detergent solution, the antibodies comprised in the immunoadsorbant are bound to agarose and a binding buffer is used.

The invention also concerns a process for the in vitro diagnosis of an infection specifically due to a HIV-2 EHO retrovirus in a biological sample detected for the possible presence of HIV-2 EHO, comprising the following steps:

- contacting the biological sample to be tested, with one of several of the preceding antigen(s) or a composition containing the same, in conditions enabling the formation of a conjugate between said antigens and the antibodies possibly present in the biological sample,
- detecting the possibly formed immunological conjugate.

Another process according to the invention is a process for the in vitro diagnosis of an infection related to HIV-2 ROD or HIV-2 EHO subtype in a biological sample detected for the possible presence of antibodies against antigens of HIV-2 ROD or of HIV-2 EHO, comprising the following steps :

- contacting the biological sample to be assayed, with an antigen of HIV-2 EHO, or a composition of the antigens, in combination with the corresponding antigens of HIV-2 ROD which show some immunological differences with those of HIV-2 EHO as explained here above, in conditions enabling the formation of a conjugate between said antigens and the antibodies possibly present in the biological sample,
- detecting the possibly formed immunological conjugate.

A kit intended to be used according to the process for the in vitro diagnosis of a retrovirus of the HIV-2 EHO subtype in a biological sample detected for the presence of antibodies against HIV-2 EHO is characterised in that it comprises :

- an antigen having the properties defined above or a composition of these antigens possibly labeled,
- means enabling the reaction of formation of the immunological complex between the above antigens and the antibodies possibly present in the biological sample to be tested, when appropriate one or several incubation buffers,
- a negative control,
- means for the detection of the possibly formed immunological complex between said antigens and the antibodies possibly present in the sample.

Another kit for the diagnosis, is a kit for the in vitro diagnosis of an infection due to a retrovirus HIV-2 ROD or HIV-2 EHO in a biological sample detected for the presence of antibodies against HIV-2 ROD or HIV-2 EHO, characterised in that it comprises :

- antigens of HIV-2 EHO, as defined in the preceding pages in combination with the corresponding antigens of HIV-2 ROD, which present different immunological properties,
- means enabling the reaction for the formation of the immunological complex between the above antigens and the antibodies possibly present in the biological sample to be tested, when appropriate

5

one or several incubation buffers,
- a negative control,
- means for the detection of the possibly formed immunological complex between said antigens and the antibodies possibly present in the sample.

The step of detection of the possibly formed immunological complex can be made by conventional methods such as Western blot or ELISA.

Preferred compositions of antigens which enable the diagnosic of HIV-2 according to the invention are for example those comprising a mixture of one or several antigens chosen among gp300, gp140, gp125, gp80 or gp36 of HIV-2 ROD with one or several antigens chosen among gp270, gp100, gp80 or gp36 of HIV-2 EHO.

Further advantages of the invention will appear in the examples and figures which follow.

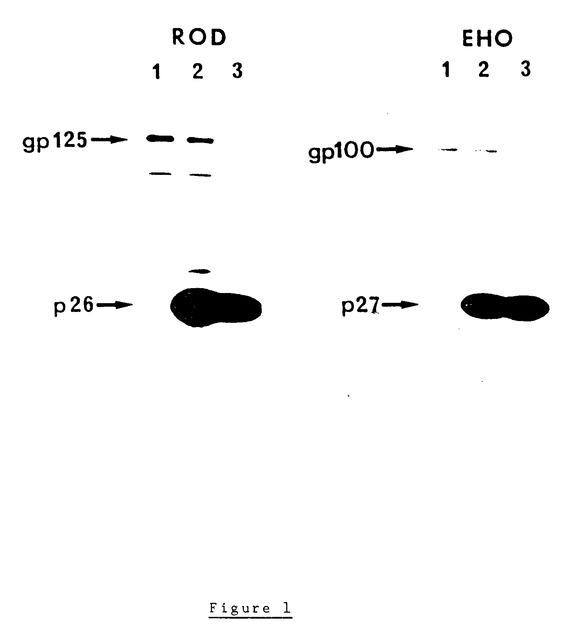

FIGURE 1 : Identification of HIV-2 EHO proteins by immunoprecipitation.

HIV-2 ROD or EHO infected CEM cells were labeled with [$^{35}$S] methionine and the culture supernatant was used in an immunoprecipitation assay using serum from the patient EHO (lanes 1), a typical HIV-2 positive serum (lanes 2) and a typical HIV-1 positive serum (lanes 3). Samples were analyzed by polyacrylamide gel (12,5%) electrophoresis. A fluorograph is shown. gp125 and p26 refer to the extracellular envelope glycoprotein and the major core protein of HIV-2 ROD, respectively. gp100 and p27 refer to the extracellular envelope glycoprotein and the major core protein of HIV-2 EHO, respectively.

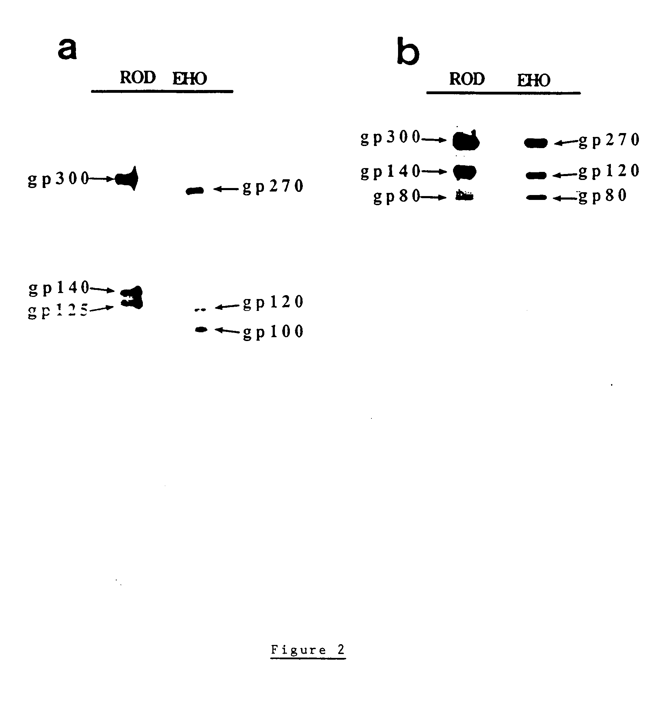

FIGURE 2 : Characterization of HIV-2 EHO envelope glycoproteins.

a/ HIV-2 ROD and EHO infected cells were labeled with [$^{35}$S] methionine and extracts were then used in an immunoprecipitation assay using a typical HIV-2 positive serum. The samples were analyzed by 5% polyacrylamide gel containing 0,1% bis-acrylamide.
b/ Extracts from HIV 2 infected cells labeled with [$^{3}$H] glucosamine were assayed by immunoprecipitation using a monoclonal antibody specific for the transmembrane glycoprotein of HIV-2 ROD. The samples were analyzed by polyacrylamide gel (10%) electrophoresis.

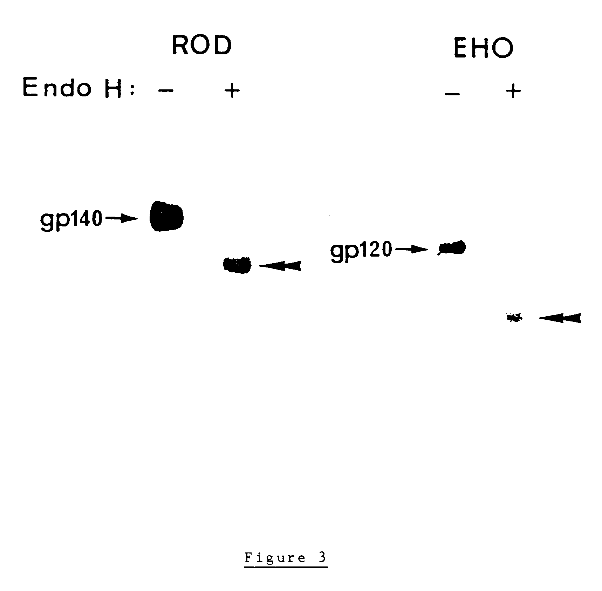

FIGURE 3 : Electrophoretic mobilities of deglycosylated envelope precursors of HIV-2 ROD and EHO.

[$^{35}$S] methionine labeled gp300 (ROD) and gp270 (EHO) were purified by immunoprecipitation followed by preparative gel electrophoresis ("Materials and Methods"). The lyophilized samples were suspended in the endo H buffer (which results in the dissociation of dimers) containing 150mM sodium citrate pH 5.5, 0.1% SDS (w/v) and 0.5mM PMSF. These samples were incubated (30°C 2h) in the absence (lanes-) or presence (lanes +) of 10 units of endo H. Reactions were stopped by the addition of 2 fold concentrated electrophoresis sample buffer. Samples were analyzed by polyacrylamide gel (7.5%) electrophoresis. Fluorographs are presented. On the right the arrows indicate the position of the digested products, 80 and 60 kDa for ROD and EHO, respectively.

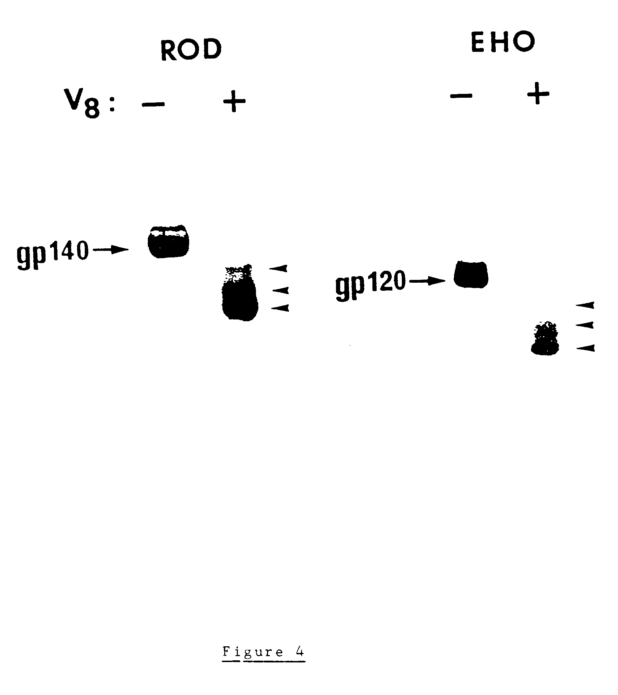

FIGURE 4 : Partial digestion of HIV-2 ROD and EHO envelope precursors with the V8 protease.

[$^{35}$S] methionine labeled envelope precursors from HIV-2 ROD and EHO were purified as described in Figure 3. The lyophilized samples were suspended in 100 $\mu$l of buffer containing 62.5mM Tris-HCl pH 6.8 and 0.1% SDS (w/v). The samples were then incubated (37°C, 30 min) without (lanes -) or with (lanes +) 1$\mu$g of staphyloccocus aureus V8 protease. Reactions were stopped by the addition of two fold concentrated electrophoresis sample buffer. The different samples were analyzed by electrophoresis in a 7.5% polyacrylamide gel. A fluorograph is shown. On the right the arrows indicate the position of the digestion products : 130, 110 and 80 kDa for gp140 ROD whereas 90, 75 AND 60 kDa for gp120 EHO.

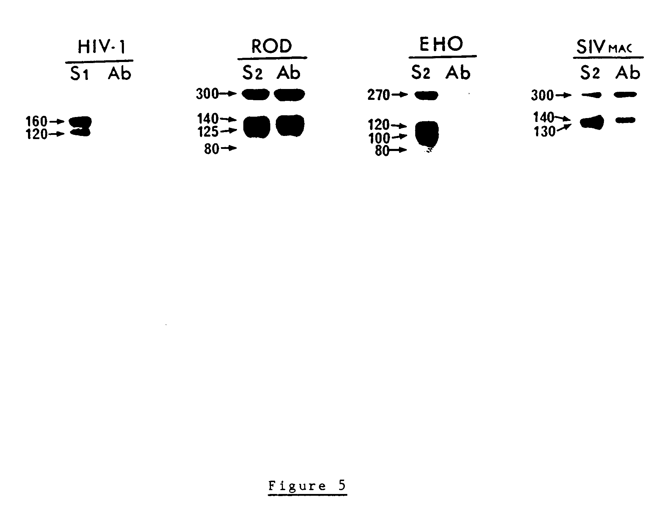

FIGURE 5 : Immunoprecipitation assay using polyclonal antibodies raised against gp300 of HIV-2 ROD.

[$^{3}$H] glucosamine labeled extracts from HIV-1 BRU, HIV-2 ROD, HIV-2 EHO and SIV mac infected cells were assayed by immunoprecipitation using a typical human HIV-1 positive serum (lane S1), a typical human HIV-2 positive serum (lanes S2) and murine polyclonal antibodies raised against gp300 of HIV-2 ROD (lanes Ab). Samples were analyzed by polyacrylamide gel (12.5%) electrophoresis. Fluorographs are shown.

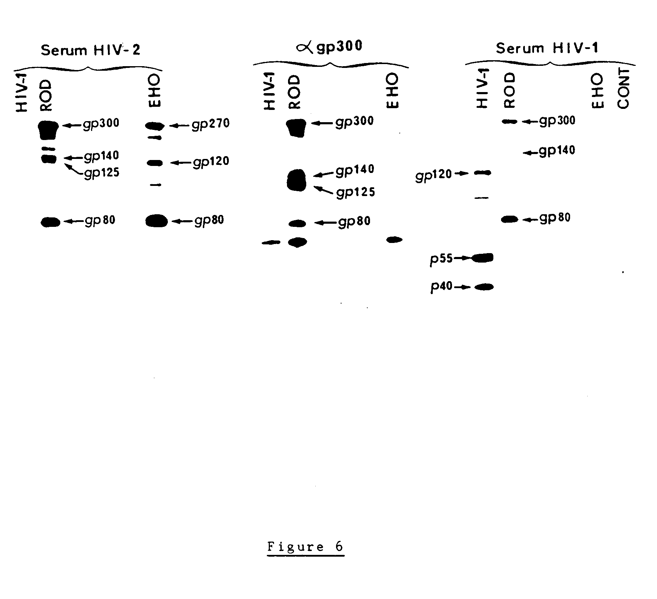

FIGURE 6 : Envelope glycoproteins of HIV-2 EHO are not recognized by anti-gp300 antibodies.

Extracts from CEM cells infected with HIV-1 or HIV-2 ROD or HIV-2 EHO were analyzed by immunoblotting using the serum of the patient EHO (section Serum HIV-2), murine anti-gp300 antibodies (section anti-gp300) and a typical HIV-1 positive serum (section Serum HIV-1). CONT refers to extracts from uninfected cells ; Polyacrylamide gel electrophoresis was carried out in 10% acrylamide (in sections Serum HIV-2 and Serum HIV-1) or 7.5% polyacrylamide (sections anti-gp300) gels. Autoradiographs are presented.

## MATERIALS AND METHODS

### MATERIALS

L-[$^{35}$S] Methionine (specific activity > 1000 $\mu$Ci/mmol), D-[6-$^3$H] Glucosamine (specific activity : 20-40 $\mu$Ci/mmol) were purchased from Amersham (Amersham, UK). Endo-$\beta$-N-acetylglucosaminidase H and Staphylococcus V8 protease were from Calbiochem, San Diego, U.S.A. Poly(A).poly(U) was obtained at Institut Curie, Paris, and prepared according to the method described by Hovanessian et al. 1982 (Journal Interferon Research vol. 2 p. 209 à 216).

The monoclonal antibody specific for the transmembrane glycoprotein was obtained from Oncogen Seattle, U.S.A. The preparation of this monoclonal antibody (referred to as mAb1H8) is described.

### VIRUS AND CELLS

HIV-1$_{BRU}$ isolate of the human immunodeficiency virus type 1 (Montagnier et al. 1984), HIV-2ROD isolate of the human immunodeficiency virus type 2 (Clavel et al., 1986) and Simian immunodeficiency virus, SIVmac$_{142}$ (Daniel et al., 1985) were used in this study.

The different cell lines and human lymphocytes were cultured in suspension medium RPMI-1640 (GIBCOBRL, Cergy-Pontoise France) containing 10% (v/v) fetal calf serum ; 2$\mu$g/ml polybrene (Sigma) was added for HIV infected cell cultures. CEM clone 13 cells are derived from the human lymphoid cell line CEM (ATCCCCL 119) and express the T4 antigen to a high level. Five days after infection with HIV-1$_{BRU}$ or HIV-2$_{ROD}$ isolates, about 80-90% of the cells produce viral particles and can be identified by a cytopathic effect corresponding to vacuolisation of cells and appearance of small syncitia.

The HUT-78 cell line is another human T4 positive lymphoid cell line that is highly permissive for the replication of SIVmac$_{142}$ (Daniel et al., 1985). Peripheral blood lymphocytes from healthy blood donors were stimulated for three days with 0,2% (w/v) phytohemagglutinin (PHA) fraction P (Difco, Detroit, USA) in RPMI-1640 medium supplemented with 10% fetal calf serum. Cells were then cultured in RPMI-1640 medium containing 10% (v/v) T cell growth factor (TCGF, Biotest). After infection with HIV-2, lymphocytes were cultured in presence of 10% (v/v) TCGF and 2$\mu$g/ml Polybrene.

The HIV-2 EHO isolate was obtained from blood lymphocytes of Ivory Coast patient (EHO) with AIDS. Blood lymphocytes were stimulated with PHA and cocultured with PHA-stimulated, normal human lymphocytes. These cultures were maintained in the presence of TCGF. The production of virus was monitored by cytopathic effects and by the reverse transcriptase activity in the supernatant (Rey et al. 1987). The virus (HIV-2 EHO) was then cultured on CEM clone 13 cells. Four to five days after infection with HIV-2 EHO approximately more than 80% of cells produced viral particles.

### METABOLIC LABELING OF CELLS

For metabolic labeling of proteins, infected cells were incubated for 16 hours at 37°C in MEM culture (Milieu Minimum Essentiel, abréviation de Minimum Essential Medium) without L-methionine and serum but supplemented with 200 $\mu$Ci/ml[$^{35}$] methionine. For metabolic labeling of glycoproteins, infected cells were incubated for 16 hours at 37°C in MEM culture medium lacking serum and glucose but supplemented with 200 $\mu$Ci/ml [$^3$H]glucosamine.

### CELLS AND VIRAL EXTRACTS

Cell pellets corrresponding to 10$^7$ cells were resuspended in 100 $\mu$l of buffer : 10mM Tris-HCl pH 7.6, 150mM NaCl, 1mm EDTA, 0.2mM PMSF, 100 units/ml aprotinin (Iniprol®, Choay) before addition of 100 $\mu$l of the same buffer containing 2% (v/v) Triton X-100®. Cells extracts were centrifuged at 12,000g for 10 minutes, and the supernatant was stored at -80°C until used. For viral extract preparations, 100$\mu$l of 10X

lysis buffer (100mM Tris-HCl pH 7.6, 1.5M NaCl, 10mM EDTA, 10% 5v/v) Triton X-100®, 100 units/ml aprotinin) was added per ml of clarified supernatant from infected CEM cells and processed as above. For the preparation of extracts from virus pellets, culture medium from infected cells was first centrifuged at 12,000g for 10 minutes before high speed centrifugation at 100,000g for 15 min in a Beckman TL100 centrifuge. Virus pellets (material from $10^7$ cells) were then solubilized in 200$\mu$l of lysis buffer.

PREPARATIVE ELCTROPHORESIS

HIV-2 glycoproteins purified by immunoprecipitation were resolved by polyacrylamide gel electrophoresis as previously described (Rey et al. 1989 J.Virol 63, 647-658) and the regions of the gel containing the viral glycoproteins were cut out by reference to the position of prestained molecular weight protein markers (BRL).

Glycoproteins were eluted by incubation for 16 hours at 4°C in elution buffer (0.1M $NaHCO_3$, 0.5mM EDTA, 0.05% (w/v) SDS, 0.2mM PMSF). The glycoprotein fractions thus obtained were lyophilized and kept refrigerated until used.

PREPARATION OF MURINE POLYCLONAL ANTIBODIES, ANTI-gp300

HIV-2 ROD envelope glycoprotein gp300 was purified from extracts of infected CEM cells (3 x $10^8$ cells) by immunoaffinity chromatography on the HIV-2 serum-Sepharose® and followed by preparative gel electrophoresis (Rey et al. 1989a précité). The purified preparation of gp300 was dissolved in 10 ml of 150mM NaCl containing 0.5M urea and 1 mg/ml of mouse serum proteins and dialyzed for 24 hours against the solution containing 150 mM NaCl and 0.5M urea. The dialyzed material was then centrifuged and 2ml aliquots were stored at -80°C. Five mice (6 weeks old) were injected intraperitoneally, five times at 12 days interval with 350 $\mu$l of the gp300 preparation (about 0.1 $\mu$g of gp300). Poly(A) Poly (U) (200 $\mu$g ; 1 mg/ml in 150 mM NaCl) was used as an adjuvant which was administered intravenously during each immunization. Two days after the last injection mice were injected intraperitoneally with a suspension of $10^6$ sarcoma 180/TG cells to prepare hyperimmune ascitic fluid (Hovanessian et al. 1988 Immunology Today 9, 161-162). Eight days later mice were sacrificed and the ascitic fluids were collected. Ascitic cells were removed by centrifugation (200g, 5 min) and the peritoneal fluid was collected.

PRODUCTION AND CHARACTERIZATION OF MONOCLONAL, mAb 1H8

HIV-2 ROD virions were cultivated in CEM cells and purified from concentrated culture supernatants by banding in sucrose gradients. Purified virus was disrupted in 0.5% Triton X-100®, 150 mM NaCl, 50mM Tris, pH 8.0, 1% aprotinin (Sigma) and clarified by ultra-centrifugation. The viral extract was then passed over a Lentil-Lectin Sepharose® 4B affinity column (Pharmacia), the column washed, and the bound glycoproteins eluted with 0.5 M methyl $\alpha$-D-mannopyranoside (Sigma), and dialyzed overnight against phosphate-buffered saline. BALB-C mice were immunized intraperitoneally with 0.3 ml of purified glycoproteins (2-5$\mu$g) reattached to Lentil-Lectin Sepharose® 4B (50-100 $\mu$l). The mice were boosted every 4-6 weeks for 24 weeks with the same immunogen and monitored for HIV-2 virion extracts and quantitatively on the Genetic Systems HIV-2 disrupted virion EIA. Three days after the last injection, spleen cells were fused with NSI myeloma cells according to the method of Kohler and Milstein. 96-well fusion plates were screened for hybridomas secreting anti-HIV-2 antibodies using the Genetic Systems HIV-2 disrupted virion EIA. Methods for the propagation and stabilization of clones hybridomas and for ascites production have been previously described Gosting et al (J. of Clinical Microbiology 25, 845-848). Hybridoma culture supernatants were screened by RIPA and Western blot analysis. Monoclonal antibody (mAb) IH8, which reacted with the transmembrane glycoprotein, was further mapped to amino-acid sequence 579-604 within the HIV-2 transmembrane glycoprotein using a synthetic peptide based EIA. The HIV-2 amino-acid sequence 579 604 is highly conserved among all HIV-2 ans SIV isolates thus far sequenced accordingly, monoclonal antibody IH8 cross-reacts with all HIV-2 and SIV isolates thus far tested. The synthetic peptide p39′ was synthesized according to the amino-acid sequence 579-604 deduced from the nucleotide sequence of the HIV-2 ROD envelope. The amino-acid sequence of peptide p39′ is the following VTAIEKYLQDQARLNSWGCAFRQVCH.

RADIO-IMMUNOPRECIPITATION ASSAY (RIPA)

Cell or viral extracts (20μl) (material corresponding to 1 x 10$^6$ infected cells) were first diluted in two volumes of RIPA buffer [(10mM Tris-HCl pH 7.6, 150mM NaCl, 1mM EDTA, 1% Triton X-100® (v/v), 0,2% sodium deoxycholate (wt/v), 0.1% SDS (wt/v) 7 mM 2 β-mercaptoethanol, 0.2 mM PMSF, 100 units/ml of aprotinin (Iniprol®, Choay)]. Diluted extracts were incubated (45 min, 4°C) with sera or with murine polyclonal or monoclonal antibodies (2-5μl). Protein A-Sepharose® was then added and the samples were further incubated for 3 h at 4°C. These samples were washed in the RIPA buffer. Proteins recovered by immunoprecipitation were eluted by heating (95°C, 5min) in the electrophoresis sample buffer [125 mM Tris-HCl, pH 6.8, 1% SDS (wt/v), 20% glycerol (v/v), 1% 2 β-mercaptoethanol]. Eluted proteins were resolved by electrophoresis in polyacrylamide SDS gels containing O.1% bis-acrylamide instead of 0.2% (wt/v) and 2M urea.

ELECTROPHORETIC TRANSFER IMMUNOBLOT ANALYSIS:WESTERN BLOT

Proteins were subjected to analysis by polyacrylamide gel electrophoresis before being electrophoretically transferred to 0.45 μm nitrocellulose sheets (Schleicher and Schüll, Dassel, FRG) in electrode buffer (20mM Tris base, 150mM glycine, 20% methanol, v/v) as described (Burnette 1981, Anal. Biochem. 112, 195-230). The electrophoretic blots were saturated with 5% (w/v) non-fat dry milk in PBS (Johnson et al. 1984 Gene Anal. Techn. I, 3-8). They were then incubated in a sealed bag (overnight 4°C) either with HIV-1 or HIV-2 positive sera (at 1 : 100 dilution) or with mouse polyclonal or monoclonal antibodies (at 1: 200 dilution) in PBS containing 10% FCS. The sheets were subsequently washed in PBS, PBS containing 5% Nonidet P-40 and then resaturated in PBS containing nonfat milk (5%). The washed sheets were then incubated (2 hr, room temperature) in a sealed bag either with a preparation of $^{125}$I-labeled protein A (Amersham, >30mCi/mg) to reveal the human polyclonal antibodies in the HIV-1 or HIV-2 sera or with a preparation of $^{125}$I-labeled goat anti-mouse immunoglobulins (Amersham ; 2-10 μCi/ μg). The sheets were removed from the bags and washed again, dried and autoradiographed (Kodak RP Royal, X-Ray films) to 24-48 hr.

RESULTS

ISOLATIONS OF HIV-2 EHO :

HIV-2 EHO was isolated from the peripheral blood lymphocytes of an AIDS patient (EHO) from Ivory Coast. Virus was isolated by coculturing PHA-stimulated lymphocytes from the patient EHO and PHA stimulated normal human lymphocytes in the presence of TCGF. Reverse transcriptase activity in the culture supernatant was detectable at 15 days associated with a typical cytopathic effect. The virus was then propagated on CEM cells by coculturing with infected lymphocytes. Supernatants of such cultures were then used to infect CEM cells and prepare the stock of virus used in the experiments discussed here. This virus belongs to the HIV-2 retrovirus type since in a dot-blot assay its genomic RNA hybridized with a probe from HIV-2 ROD but not from HIV-1 BRU.

The stock preparation of HIV-2 EHO infects CEM cells and causes a cytopathic effect. Three to four days after infection of CEM cells with HIV-2 EHO, about 80 to 90% of cells produced viral proteins detectable by immunofluorescence studies using an HIV-2 positive serum. Four days after infection most of the cells showed a clear cytopathic effect corresponding to vacuolization of cells and appearance of small syncitia.

In order to characterize the proteins of HIV-2 EHO virus, infected cells were metabolically labeled with [$^{35}$S]methionine and the viral pellet in the culture supernatant was assayed by immunoprecipitation using different sera :

1/ the serum of the patient infected by HIV-2 EHO;

2/ a typical HIV-2 positive serum;

3/ a typical HIV-1 positive serum.

These assays were carried out in parallel with extracts of [$^{35}$S]methionine labeled HIV-2 ROD virus. The HIV-2 positive serum and the serum of the patient EHO both recognized the extracellular glycoprotein gp125 of HIV-2 ROD. However, only the HIV-2 positive serum recognized the major core protein p26 of HIV-2 ROD (Figure 1, lanes 1 and 2, ROD). The HIV-1 positive serum did not recognize gp125 but it could immunoprecipitate p26 (Figure 1, lane 3, ROD). This is in accord with previous results indicating that the gag proteins of HIV-1 and HIV-2 are antigenically cross-reactive whereas the env proteins are not (Clavel et

9

al. 1986a). The reactivity of these three sera with the viral proteins of HIV-2 EHO was similar to that of HIV-2 ROD. The serum of the patient-EHO and the HIV-2 positive serum immunoprecipitated a 100 kDa protein which was presumed to be the extracellular envelope glycoprotein of HIV-2 EHO (Figure 1, lanes 1 and 2 EHO). This 100 kDa protein was specific to HIV-2 since it was not recognized by the HIV-1 positive serum. Both HIV-1 and HIV-2 positive sera precipitated a 27 kDa protein which was probably the major core protein of HIV-2 EHO. However, this latter protein was not recognized by the serum from patient EHO (Figure 1, EHO). These observations indicate the absence of anti-gag antibodies in the serum of patient EHO. Such a phenomenon has been reported in HIV-1 infected individuals and it is associated with the developement of AIDS. The late stages of HIV-1 infection are characterized by an increased production of antigens and a decreased level of antibodies directed against the major core protein. In fact this observation is routinely used as a marker to monitor the switch from latent to active HIV infection (Lange et al., 1986 Br. Med. J.293, 1459-1462; Pedersen et al. , 1987 Br. Med. J. 295, 567-572).

## IDENTIFICATION OF EXTERNAL AND TRANSMEMBRANE ENVELOPE PROTEINS OF HIV-2 EHO

In these experiments, HIV-2 ROD and EHO infected CEM cells were metabolically labeled with [$^{35}$S]-methionine and the labeled extracts were assayed by immunoprecipitation using different types of antibodies : a typical HIV-2 positive serum which identifies envelope proteins of HIV-2 ROD (Rey et al. 1989a), a monoclonal antibody specific for the transmembrane glycoprotein of HIV-2 ROD, a monoclonal antibody specific for the major core protein p26 of HIV-2 ROD and rabbit polyclonal antibodies specific for the vpx protein.

In the case of HIV-2 ROD envelope glycoproteins, it has been previously shown that the envelope precursor (gp140) forms a dimer (gp300) during its processing, required for the production of mature envelope proteins : the extracellular glycoprotein gp125 and the dimeric form of the transmembrane glycoprotein gp80. The monomer of the transmembrane glycoprotein is rarely detectable under the experimental conditions used in our experiments. In order to compare the molecular weight of the envelope glycoproteins an electrophoresis was performed in a 5% polyacrylamide gel containing 0.1% bis-acrylamide instead of 0.2%. Figure 2a shows the results of this experiment indicating that the envelope precursor, the dimeric form of the envelope precursor and the extracellular envelope glycoprotein are 20-30kDa smaller in HIV-2 EHO compared to HIV-2 ROD. In order to identify the transmembrane glycoprotein of HIV-2 EHO we used a monoclonal antibody mab1H8 against the transmembrane glycoprotein of HIV-2 ROD. As it is expected, this monoclonal antibody identifies also the envelope precursor and its dimeric form. Accordingly, the monoclonal antibody immunoprecipitated gp120, gp270 and gp80 from HIV-2 EHO infected cells compared to gp140, gp300 and gp80 from HIV-2 ROD infected cells (Figure 2b). The electrophoretic mobility of the transmembrane glycoprotein dimer of HIV-2 EHO was slightly slower than that of HIV-2 ROD. Since this difference is not significant, for convenience we will refer to it as gp80. Pulse-chase experiments and studies on the kinetics of accumulation of envelope proteins of HIV-2 EHO, indicated that gp120 was the first glycoprotein synthetized before the formation of the precursor dimer gp270 and the production of mature envelope proteins : extracellular glycoprotein gp100 and the transmembrane glycoprotein dimer gp80. Glycosylation of all envelope proteins was blocked by tunicamycin, an antibiotic which inhibits N-linked glycosylation of proteins (Schwartz et al. 1976 J.Virol. 19, 782-791 ; Kornfeld and Kornfeld 1985, Ann.Rev.Biochem. 54, 631-664). Among these glycoproteins of the envelope only gp100 and gp80 were found to be associated with virus particles according to previously reported results on HIV-2 ROD (Rey et al., 1989a).

## CHARACTERIZATION OF HIV-2 ROD AND HIV-2 EHO ENVELOPE PRECURSORS

There are at least 30 potential N-linked glycosylation sites on the envelope precursor of HIV-2 ROD. In view of this, it remained possible that the 20 kDa difference between the envelope precursor of HIV-2 EHO and ROD might be due to a reduced number of oligosaccharide chains in the envelope precursor of HIV-2 EHO. For this reason, the electrophoretic mobilities of these precursors was analysed after deglycosylation by β-N-acetylglucosaminase H (endo H), which cleaves high mannose type oligosaccharide chains (Tarentino et al., 1974 H.J.Biol. Chem. 249, 818-824). HIV-2 EHO or ROD infected cells were labeled with [$^{35}$S] methionine and cell extracts were used for the purification of the dimeric forms of envelope precursors by immunoprecipitation and preparative gel electrophoresis. The purified dimer precursors were then dissociated in the presence of SDS and acidic pH (Rey et al., 1989a) before digestion with endo H. the apparent molecular weights of the envelope precursors gp140 (ROD) and gp120 (EHO) were reduced to proteins of 80 and 60 kDa, respectively (Figure 3). These results therefore indicated that the 20 kDa difference between the envelope precursors of the two HIV-2 isolates was not due to a difference in the number of N-linked oligosaccharide chains. Under the experimental conditions of these experiments, digestion with endo-H resulted in a complete cleavage of all oligosaccharide chains.

In order to compare the polypeptide pattern of the envelope precursors of HIV-2 ROD and EHO, we carried out partial proteolysis experiments with <u>Staphyloccocus aureus</u> V8 protease, which manifests specificity for glutamyl bonds. The purified envelope-dimer precursors were first dissociated, before partial proteolysis with different concentrations of the V8 protease. The results obtained with 1μg of the protease are shown in Figure 4. The gp 140 arising from the dissociation of gp300 of HIV-2 ROD was converted to three major polypeptides of 130, 110 and 80 kDa whereas, the gp120 arising from the dissociation of gp270 of HIV-2 EHO was converted to a major 60 kDa polypeptide and two faint 90 and 75 kDa polypeptides. Thus digestion of gp140 and gp120 with the V8 protease resulted in the production of distinct polypeptide patterns which reflected differences in the amino acid composition between these two envelope precursors.

## HIV-2 EHO ENVELOPE GLYCOPROTEINS ARE NOT RECOGNIZED BY MURINE POLYCLONAL ANTIBODIES AGAINST THE ENVELOPE GLYCOPROTEINS OF HIV-2 ROD.

Polyclonal antibodies were prepared by immunizing mice with a purified preparation of gp300 from cells infected with HIV-2 ROD ("Materials and Methods"). These antibodies were referred to as anti-gp300 polyclonal antibodies. The reactivity of these antibodies was tested in an immunoprecipitation assay using extracts from HIV-1 BRU, HIV-2 ROD, HIV-2 EHO and SIV-mac infected cells labeled with [3H] glucosamine (Figure 5). These assays were carried out in parallel with typical human sera specific for HIV-1 and HIV-2. Anti-gp300 polyclonal antibodies immunoprecipitated gp300, gp140, gp125 and gp80 of HIV-2 ROD with a similar affinity as the HIV-2 positive human serum. In contrast, anti-gp300 antibodies did not recognize any of the envelope glycoproteins of HIV-2 EHO (Figure 5, sections ROD and EHO). However, these latter (gp270, gp120, gp100 and gp80) were immunoprecipitated by the typical HIV-2 positive human serum. These observations indicate the presence of similar and some distinct epitopes on both the extracellular and the transmembrane regions of the envelope glycoproteins of HIV-2 EHO (Figure 5, sections ROD AND EHO).

Anti-gp300 antibodies did not recognize HIV-1 envelope glycoproteins. The precursor gp160 and the extracellular glycoprotein gp120, which as expected, were immunoprecipitated by the HIV-1 specific human serum (Figure 5, section HIV-1). Interestingly, anti-gp300 antibodies immunoprecipitated SIV-mac envelope glycoproteins : the envelope precursor gp140, the precursor dimer gp300 and the extracellular glycoprotein gp130. These SIV-mac glycoproteins were also immunoprecipitated by the HIV-2 specific human serum (Figure 5 section SIV-mac). The reactivity of anti-gp300 antibodies with envelope glycoproteins of both HIV-2 ROD and SIV-mac but not with those of HIV-2 EHO, confirms the important homology between HIV-2 ROD and SIV-mac and also suggests that HIV-2 EHO belongs to a subtype of the HIV-2 that could be differentiated by its lower homology to both HIV-2 ROD and SIV-mac.

Anti-gp300 antibodies along HIV-2 and HIV-1 specific human sera were used in an immunoblot assay with extracts from HIV-1 BRU, HIV-2 ROD and HIV-2 EHO infected cells (Figure 6). The HIV-2 positive serum identified strongly gp300, gp140 and gp80 of HIV-2 ROD, and gp270, gp120 and gp80 of HIV-2 EHO but it showed no reactivity with envelope glycoproteins of HIV-1 (Figure 5, section Serum HIV-2 ; 10% polyacrylamide gel). The poor affinity of this serum with the extracellular glycoprotein of HIV-2 was probably due to low reactivity with the denatured protein since this same serum could identify gp125 (ROD) and gp100 (EHO) under native conditions of the immunoprecipitation assay (Figure 5). Anti-gp300 antibodies identified strongly all the envelope glycoprotein of HIV-2 ROD (gp300, gp140, gp125 and gp80) but it showed no reactivity with the corresponding envelope glycoproteins of HIV-2 EHO nor with proteins of HIV-1 (Figure 6, section anti-gp300 ; 7.5% polyacrylamide gel). The reactivity of anti-gp300 antibodies with the 60kDa protein was found to be not specific because it was observed in cell extracts independent of virus infection. These results indicate that anti-gp300 antibodies do not react with envelope glycoproteins of HIV-2 EHO, either native or denatured form.

Comparison of the results obtained by immunoblot analysis and the immunoprecipitation assays (Figures 5 and 6), show that patient serum and anti-gp300 polyclonal antibodies recognize the denatured form of gp80 better than its native form. The native form of this transmembrane glycoprotein-dimer probably has a conformation which masks the epitopes identified by these different antibodies.

THE CROSS-REACTIVITY OF AN HIV-1 POSITIVE SERUM WITH DENATURED ENVELOPE GLYCOPROTEINS OF HIV-2 ROD BUT NOT HIV-2 EHO.

In these experiments, an HIV-1 positive human serum was used, which in an immunoprecipitation assay manifested a specificity towards HIV-1 extracellular envelope glycoprotein gp120. In an immunoblot assay, this serum identified gp120 and gag precursors p55 and P40. In addition, this HIV-1 positive serum reacted strongly with gp300 and gp80 of HIV-2 ROD whereas no reactivity was observed with HIV-2 EHO envelope glycoproteins (Figure 6, section Serum HIV-1 ; 10% polyacrylamide gel). This observation indicates that there might be some cross-reactivity between few HIV-1 positive sera and HIV-2 envelope glycoproteins. This cross-reactivity seems to be directed towards the dimeric forms of the envelope, i.e., the dimeric form of the envelope precursor gp300 and the transmembrane glycoprotein dimer gp80. The monomeric form of the precursor gp140 was very weakly recognized by this HIV-1 positive serum, whereas the extracellular glycoprotein gp125 was not at all recognized. In view of these, it is possible to suggest that HIV-1 positive serum recognized an epitope in the transmembrane region of the HIV-2 ROD envelope glycoprotein and for this interaction both denaturation and dimerization were required. These results emphasize antigenic differences between the envelope glycoproteins of HIV-2 ROD and EHO.

The following table 1 gives the results of the comparison between the different retroviruses HIV-1 and HIV-2 and isolate of the latter.

## Table 1 : Comparative molecular weight (kDa) of viral proteins of HIV-1 BRU, HIV-2 ROD, HIV-2 EHO, SIVmac

| VIRUS | ENV | | | | | GAG | | VPX |
| | Pr | | EC gp | TM gp | | Pr | M P | X |
| | Monomer | Dimer | | Monomer | Dimer | | | |
|---|---|---|---|---|---|---|---|---|
| HIV-1 BRU | 160 | – | 120 | 41 | – | 55 | 25 | – |
| HIV-2 ROD | 140 | 300 | 125 | 36 | 80 | 55 | 26 | 16 |
| HIV-2 EHO | 120 | 270 | 100 | 36 | 80 | 56 | 27 | 14 |
| SIV mac | 140 | 300 | 130 | 32 | 65 | 56 | 28 | 14 |

Pr : Precursor
EC gp : Extracellular glycoprotein
TM gp : Transmembrane glycoprotein
MP : Major Protein of the core

**Claims**

1. Purified antigen of the human HIV-2 EHO retrovirus characterised in that it is constituted by the envelope glycoprotein of HIV-2 EHO or a glycoprotein of a variant thereof or part of said antigen, or an antigen having the same immunological properties, which antigen is recognizable by human HIV-2 positive serum which is capable of recognizing the antigens of HIV-2 ROD in an immunoprecipitation assay or in a western blot assay and it is not recognized by antibodies directed against the glycoprotein gp300 of HIV-2 ROD in an immunoprecipitation assay or in a western blot assay under the same conditions.

**2.** Purified antigen of the HIV-2 EHO retrovirus or a glycoprotein having the same immunological properties and obtained from a variant of the HIV-2 EHO retrovirus according to claim 1, characterised in that it is encoded by the <u>env</u> gene and in that it has a molecular weight of the order of 120 kDa corresponding to the envelope precursor.

**3.** Purified antigen of the HIV-2 EHO retrovirus or a glycoprotein having the same immunological properties and obtained from a variant of the HIV-2 EHO retrovirus according to claim 1, characterised in that it is a glycoprotein gp270 corresponding to a dimeric form of the gp120 and in that it has a molecular weight of the order of 270 kDa.

**4.** Purified antigen of the HIV-2 EHO retrovirus or a glycoprotein having the same immunological properties and obtained from a variant of the HIV-2 EHO retrovirus according to claim 1, characterised in that it corresponds to the mature product of the precursor gp120 and in that it is the extracellular envelope glycoprotein gp100 having a molecular weight of the order of 100 kDa.

**5.** Purified antigen or a glycoprotein having the same immunological properties and obtained from a variant of HIV-2 EHO according to claim 1, characterised in that it is a glycoprotein gp36, having a molecular Weight of the order of 36 kDa.

**6.** Purified antigen or a glycoprotein having the same immunological properties, and obtained from a variant of HIV-2 EHO according to claim 1, characterised in that it is a glycoprotein gp80 and it corresponds to the dimeric form of the gp36 according to claim 5.

**7.** Purified antigen of the HIV-2 EHO retrovirus or an unglycosylated product of the glycoprotein having the same immunological properties and obtained from a variant of the HIV-2 EHO retrovirus characterised in that it is a protein p60 having the same amino acid composition as the glycoprotein gp120 according to claim 2 and in that it has no oligosaccharide chains.

**8.** Composition for the <u>in vitro</u> diagnosis of an infection especially due to a human retrovirus of the HIV-2 EHO subtype, characterised in that it comprises at least one antigen according to anyone of claims 1 to 7.

**9.** Composition according to claim 8, characterised in that it contains the precursor gp120 of the envelope glycoprotein, having a molecular weight of the order of 120 kDa.

**10.** Composition according to claim 8, characterised in that it further contains the extracellular envelope glycoprotein gp100, having a molecular weight of the order of 100 kDa.

**11.** Composition according to claim 8, characterised in that it further contains the dimeric glycoprotein gp270, having a molecular weight of the order of 270 kDa.

**12.** Polyclonal antibodies produced in animals, characterised in that they recognize a purified antigen according to anyone of claims 1 to 7 and in that they do not recognize the envelope glycoproteins of HIV-2 ROD or the envelope glycoproteins of HIV-1.

**13.** Monoclonal antibodies except an antibody which recognizes specifically an epitope located within the amino acid sequence of the transmembrane glycoprotein of HIV-2 ROD comprised between amino acid residues at positions 579 and 604, characterised in that they recognize an epitope which is common to the purified antigens according to claims 2 to 4 and which is not recognized by antibodies directed against the gp300 or by antibodies directed against one of the envelope related gp140 or gp125 of HIV-2 ROD retrovirus subtype.

**14.** Monoclonal antibodies except an antibody which recognizes specifically an epitope located within the amino acid sequence of the transmembrane glycoprotein of HIV-2 ROD comprised between amino acid residues at positions 579 and 604, characterised in that they recognize specifically the glycoprotein gp100 according to claim 4, said epitope being not recognized by antibodies directed against the gp300 or by antibodies directed against one of the gp140 or gp125 of HIV-2 ROD subtype.

**15.** Monoclonal antibodies except an antibody which recognizes specifically an epitope located within the amino acid sequence of the transmembrane glycoprotein of HIV-2 ROD comprised between amino acid residues at positions 579 and 604, characterised in that they recognize specifically the glycoprotein gp120 according to claim 2, said epitope being not recognized by antibodies directed against the gp300 or by antibodies directed against one of the gp140 or gp125 of HIV-2 ROD subtype.

**16.** Monoclonal antibodies characterised in that they recognize specifically the glycoprotein gp270 according to claim 3, said epitope being not recognized by antibodies directed against the gp300 or by antibodies directed against one of the gp140 or gp125 of HIV-2 ROD subtype.

**17.** Kit for the in vitro diagnosis of an infection due to a retrovirus of the HIV-2 EHO subtype in a biological sample to be assayed for the presence of antibodies against HIV-2 EHO characterised in that it comprises:
- an antigen according to anyone of claims 1 to 7 or a composition of antigens according to anyone of claims 8 to 11, possibly labelled,
- means enabling the reaction for the formation of the immunological complex formed between the above antigens and the antibodies if said antibodies are present in the biological sample to be tested, when appropriate one or several incubation buffers,
- a negative control,
- means for the detection of the immunological complex formed between said antigens and the antibodies if said antibodies are present in the sample,

**18.** Kit for the in vitro diagnosis of an infection due to a retrovirus HIV-2 ROD or HIV-2 EHO in a biological sample to be assayed for the presence of antibodies against HIV-2 ROD or HIV-2 EHO, characterised in that it comprises:
- antigens of HIV-2 EHO, according to anyone of claims 1 to 7, in combination with the corresponding antigens fo HIV-2 ROD which present different immunological properties,
- means enabling the reaction for the formation of the immunological complex between the above antigens and the antibodies possibly present in the biological sample to be tested, when appropriate one or several incubation buffers,
- a negative control,
- means for the detection of the immunological complex formed between said antigens and the antibodies if said antibodies are present in the sample.

**19.** Process for the preparation of a purified antigen according to anyone of claims 1 to 7, comprising the following steps:
- lysing cells infected with a human retrovirus HIV-2 EHO and separating the supernanant from the cellular extract,
- incubating the diluted extract with a serum of a patient infected with HIV-2 EHO retrovirus or with polyclonal or monoclonal antibodies according to anyone of claims 12 to 15 on an immunoaffinity column under conditions sufficient to allow the formation of a complex between said antibodies and antigens of the cellular extract,
- washing the immunoaffinity column to remove the molecules which are not retained on the support,
- eluting the antigens recovered by immunoprecipitation in an electrophoresis sample buffer,
- resolving the eluted antigens.

**20.** Process for the in vitro diagnosis of an infection specifically due to a HIV-2 EHO retrovirus in a biological sample to be assayed for the possible presence of antibodies against antigens of HIV-2 EHO, comprising the following steps:
- contacting the biological sample to be tested, with an antigen according to anyone of claims 1 to 7 or a composition according to anyone of claims 8 to 11, under conditions enabling the formation of a conjugate between said antigens and the antibodies possibly present in the biological sample,
- detecting the immunological conjugate formed, if any.

**21.** Process for the in vitro diagnosis of an infection in a biological sample to be assayed for the possible presence of antibodies against antigens of HIV-2 ROD or HIV-2 EHO, comprising the following steps:

- contacting the biological sample to be tested, with an antigen according to anyone of claims 1 to 7 or a composition according to anyone of claims 8 to 11, in combination with the corresponding antigens of HIV-2 ROD, which show some immunological differences with those of HIV-2 EHO especially regarding the reaction with antibodies against gp300, under conditions enabling the formation of a conjugate between said antigens and the antibodies possibly present in the biological sample,
- detecting the immunological conjugate formed, if any.

**Patentansprüche**

1. Gereinigtes Antigen des humanen HIV-2 EHO-Retrovirus, dadurch **gekennzeichnet,** dass es aus dem Hüll-Glycoprotein des HIV-2 EHO oder einem Glycoprotein einer Variante davon, oder einem Teil des Antigens oder einem Antigen mit denselben immunologischen Eigenschaften besteht, wobei das Antigen durch ein humanes HIV-2-positives Serum erkennbar ist, das zur Erkennung von Antigenen des HIV-2 ROD in einem Immunpräzipitationstest oder einem Western-Blott-Test in der Lage ist, und das nicht von gegen das Glycoprotein gp300 von HIV-2 ROD gerichteten Antikörpern in einem Immunpräzipitationstest oder einem Western-Blott-Test unter den gleichen Bedingungen erkannt wird.

2. Gereinigtes Antigen des HIV-2 EHO-Retrovirus oder ein Glycoprotein mit den gleichen immunologischen Eigenschaften, erhalten aus einer Variante des HIV-2 EHO-Retrovirus nach Anspruch 1, dadurch **gekennzeichnet,** dass es von dem env-Gen codiert wird, und dass es ein Molekulargewicht in der Grössenordnung von 120 kDa, entsprechend dem Hüllvorläufer, hat.

3. Gereinigtes Antigen des HIV-2 EHO-Retrovirus oder Glycoprotein mit den gleichen immunologischen Eigenschaften, erhalten von einer Variante des HIV-2 EHO-Retrovirus nach Anspruch 1, dadurch **gekennzeichnet,** dass es das Glycoprotein gp270, entsprechend einer dimeren Form des gp120, ist, und dass es ein Molekulargewicht in der Grössenordnung von 270 kDa hat.

4. Gereinigtes Antigen des HIV-2 EHO-Retrovirus oder Glycoprotein mit den gleichen immunologischen Eigenschaften, erhalten von einer Variante des HIV-2 EHO-Retrovirus nach Anspruch 1, dadurch **gekennzeichnet,** dass es dem reifen Produkt des Vorläufers gp120 entspricht, und dass es das extrazelluläre Hüll-Glycoprotein gp100 mit einem Molekulargewicht in der Grössenordnung von 100 kDa ist.

5. Gereinigtes Antigen oder Glycoprotein mit den gleichen immunologischen Eigenschaften und erhalten von einer Variante von HIV-2 EHO gemäss Anspruch 1, dadurch **gekennzeichnet,** dass es das Glycoprotein gp36 mit einem Molekulargewicht in der Grössenordnung von 36 kDa ist.

6. Gereinigtes Antigen oder Glycoprotein mit den gleichen immunologischen Eigenschaften und erhalten von einer Variante von HIV-2 EHO nach Anspruch 1, dadurch **gekennzeichnet,** dass es das Glycoprotein gp80 ist und der dimeren Form des gp36 nach Anspruch 5 entspricht.

7. Gereinigtes Antigen des HIV-2 EHO-Retrovirus oder ein nicht-glycosyliertes Produkt des Glycoproteins mit den gleichen immunologischen Eigenschaften und erhalten von einer Variante des HIV-2 EHO-Retrovirus, dadurch **gekennzeichnet,** dass es das Protein p60 mit der gleichen Aminosäure-Zusammensetzung wie das Glycoprotein gp120 nach Anspruch 2 ist, und dass es keine Oligosaccharidketten hat.

8. Zusammensetzung zur in vitro-Diagnose einer Infektion, insbesondere aufgrund eines humanen Retrovirus vom HIV-2 EHO-Subtyp, dadurch **gekennzeichnet,** dass sie mindestens ein Antigen nach einem der Ansprüche 1 bis 7 umfasst.

9. Zusammensetzung nach Anspruch 8, dadurch **gekennzeichnet,** dass sie den Vorläufer gp120 des Hüll-Glycoproteins mit einem Molekulargewicht in der Grössenordnung von 120 KDa enthält.

10. Zusammensetzung nach Anspruch 8, dadurch **gekennzeichnet,** dass sie ferner das extrazelluläre Hüll-Glycoprotein gp100 mit einem Molekulargewicht in der Grössenordnung von 100 kDa enthält.

**11.** Zusammensetzung nach Anspruch 8, dadurch **gekennzeichnet,** dass sie ferner das dimere Glycoprotein gp270 mit einem Molekulargewicht in der Grössenordnung von 270 kDa enthält.

**12.** Polyklonale Antikörper, erzeugt in Tieren, dadurch **gekennzeichnet,** dass sie ein gereinigtes Antigen nach einem der Ansprüche 1 bis 7 erkennen, und dass sie nicht die Hüll-Glycoproteine von HIV-2 ROD oder die Hüll-Glycoproteine von HIV-1 erkennen.

**13.** Monoklonale Antikörper, mit Ausnahme eines Antikörpers, der spezifisch ein innerhalb der Aminosäuresequenz des transmembranen Glycoproteins von HIV-2 ROD lokalisiertes Epitop, das zwischen den Aminosäureresten an den Positionen 579 und 604 enthalten ist, erkennt, dadurch **gekennzeichnet,** dass sie ein Epitop erkennen, das den gereinigten Antigenen gemäss Ansprüchen 2 bis 4 gemeinsam ist, und das nicht durch Antikörper erkannt wird, die gegen gp300 gerichtet sind, oder von Antikörpern erkannt wird, die gegen eines der die Hülle betreffenden gp140 oder gp125 vom HIV-2 ROD-Retrovirus-Subtyp gerichtet sind.

**14.** Monoklonale Antikörper, mit Ausnahme eines Antikörpers, der spezifisch ein Epitop erkennt, das innerhalb der Aminosäuresequenz des transmembranen Glycoproteins von HIV-2 ROD lokalisiert ist, das zwischen den Aminosäureresten an den Positionen 579 und 604 enthalten ist, dadurch **gekennzeichnet,** dass sie spezifisch das Glycoprotein gp100 nach Anspruch 4 erkennen, wobei das Epitop nicht von Antikörpern erkannt wird, die gegen das gp300 gerichtet sind, oder von Antikörpern, die gegen eines von gp140 oder gp125 vom HIV-2 ROD-Subtyp gerichtet sind.

**15.** Monoklonale Antikörper, mit Ausnahme eines Antikörpers, der spezifisch ein Epitop erkennt, das innerhalb der Aminosäuresequenz des transmembranen Glycoproteins von HIV-2 ROD lokalisiert ist, die zwischen den Aminosäureresten an den Positionen 579 und 604 enthalten sind, dadurch **gekennzeichnet,** dass sie spezifisch das Glycoprotein gp120 nach Anspruch 2 erkennen, wobei das Epitop nicht durch Antikörper erkannt wird, die gegen gp300 gerichtet sind, oder durch Antikörper, die gegen eines von gp140 oder gp125 vom HIV-2 ROD-Subtyp gerichtet sind.

**16.** Monoklonale Antikörper, dadurch **gekennzeichnet,** dass sie spezifisch das Glycoprotein gp270 nach Anspruch 3 erkennen, wobei das Epitop nicht durch Antikörper erkannt wird, die gegen das gp300 gerichtet sind, oder von Antikörpern, die gegen eines von gp140 oder gp125 vom HIV-2 ROD-Subtyp gerichtet sind.

**17.** Kit zur in vitro-Diagnose einer Infektion aufgrund eines Retrovirus vom HIV-2 EHO-Subtyp in einer zu testenden biologischen Probe auf die Anwesenheit von Antikörpern gegen HIV-2 EHO, dadurch **gekennzeichnet,** dass es umfasst:
ein Antigen nach einem der Ansprüche 1 bis 7 oder eine Zusammensetzung von Antigenen nach einem der Ansprüche 8 bis 11, das möglicherweise markiert ist,
Mittel, die die Reaktion zur Bildung eines immunologischen Komplexes, gebildet zwischen den obigen Antigenen und Antikörpern, erlauben, falls die Antikörper in der zu testenden biologischen Probe vorhanden sind, falls geeignet, einer oder mehrere Inkubationspuffer,
eine negative Kontrolle,
Mittel zum Nachweis des zwischen den Antigenen und den Antikörpern gebildeten immunologischen Komplexes, falls die Antikörper in der Probe vorliegen.

**18.** Kit zur in vitro-Diagnose einer Infektion aufgrund eines Retrovirus HIV-2 ROD oder HIV-2 EHO in einer auf Anwesenheit von Antikörpern gegen HIV-2 ROD oder HIV-2 EHO zu testenden biologischen Probe, dadurch **gekennzeichnet,** dass es umfasst:
Antigene von HIV-2 EHO nach einem der Ansprüche 1 bis 7, in Kombination mit den entsprechenden Antigenen für HIV-2 ROD, die verschiedene immunologische Eigenschaften zeigen,
Mittel, die eine Reaktion unter Bildung eines immunologischen Komplexes zwischen den obigen Antigenen und den möglicherweise in der zu testenden biologischen Probe vorhandenen Antikörpern erlauben, wenn geeignet, einer oder mehrere Inkubationspuffer,
eine negative Kontrolle,
Mittel zum Nachweis des zwischen den Antigenen und Antikörpern gebildeten immunologischen Komplexes, falls die Antikörper in der Probe vorliegen.

**19.** Verfahren zur Herstellung eines gereinigten Antigens nach einem der Ansprüche 1 bis 7, umfassend die folgenden Schritte:

Lysieren von mit einem humanen Retrovirus HIV-2 EHO infizierten Zellen und Abtrennen des Überstands vom Zellextrakt,

Inkubation des verdünnten Extrakts mit einem Serum eines Patienten, der mit einem HIV-2 EHO-Retrovirus infiziert ist, oder mit polyklonalen oder monoklonalen Antikörpern nach einem der Ansprüche 12 bis 15, auf einer Immun-Affinitätssäule unter Bedingungen, die ausreichend sind für eine Bildung eines Komplexes zwischen den Antikörpern und den Antigenen des Zellextrakts,

Waschen der Immun-Affinitätssäule unter Entfernung von Molekülen, die nicht auf dem Träger zurückgehalten werden,

Elution der durch Immunpräzipitation in einem Elektrophorese-Probenpuffer gewonnenen Antigene,

Auftrennen der eluierten Antigene.

**20.** Verfahren zur in vitro-Diagnose einer Infektion, die im wesentlichen auf einem HIV-2 EHO-Retrovirus beruht, in einer zu testenden biologischen Probe auf die Anwesenheit von Antikörpern gegen Antigene von HIV-2 EHO, umfassend die folgenden Schritte:

in Kontakt bringen der zu testenden biologischen Probe mit einem Antigen nach einem der Ansprüche 1 bis 7 oder einer Zusammensetzung nach einem der Ansprüche 8 bis 11, unter Bedingungen, die die Bildung eines Konjugats zwischen den Antigenen und den möglicherweise in der biologischen Probe vorhandenen Antikörpern erlauben,

Nachweis des möglicherweise gebildeten immunologischen Konjugats.

**21.** Verfahren zur in vitro-Diagnose einer Infektion in einer auf die mögliche Anwesenheit von Antikörpern gegen Antigene von HIV-2 ROD oder HIV-2 EHO zu testenden biologischen Probe, umfassend die folgenden Schritte:

in Kontakt bringen der zu testenden biologischen Probe mit einem Antigen nach einem der Ansprüche 1 bis 7 oder einer Zusammensetzung nach einem der Ansprüche 8 bis 11, in Kombination mit den entsprechenden Antigenen von HIV-2 ROD, die einige immunologische Unterschiede zu jenen von HIV-2 EHO, insbesondere im Hinblick auf die Reaktion mit den Antikörpern gegen gp300 zeigen, unter Bedingungen, die die Bildung eines Konjugats zwischen den Antigenen und den möglicherweise in der Probe vorhandenen Antikörpern erlauben,

Nachweis des möglicherweise gebildeten immunologischen Konjugats.

**Revendications**

**1.** Antigène purifié du rétrovirus humain HIV-2 EHO, caractérisé en ce qu'il est constitué de la glycoprotéine d'enveloppe de HIV-2 EHO ou d'une glycoprotéine d'un variant de celui-ci, ou une partie dudit antigène, ou un antigène ayant les mêmes propriétés immunologiques, lequel antigène est reconnaissable par le sérum humain positif pour HIV-2, susceptible de reconnaître les antigènes de HIV-2 ROD dans un dosage d'immunoprécipitation ou dans un dosage de Western blot et il n'est pas reconnu par les anticorps dirigés come la glycoprotéine gp300 de HIV-2 ROD dans un dosage d'immunoprécipitation ou dans un dosage de Western blot dans les mêmes conditions.

**2.** Antigène purifié du rétrovirus HIV-2 EHO ou une glycoprotéine ayant les mêmes propriétés immunologiques et obtenue à partir d'un variant du rétrovirus HIV-2 EHO selon la revendication 1, caractérisé en ce qu'il est codé par le gène env et en ce qu'il a un poids moléculaire de l'ordre de 120 kDa correspondant au précurseur d'enveloppe.

**3.** Antigène purifié du rétrovirus HIV-2 EHO ou une glycoprotéine ayant les mêmes propriétés immunologiques et obtenue à partir d'un variant du rétrovirus HIV-2 EHO selon la revendication 1, caractérisé en ce qu'il est une glycoprotéine gp270 correspondant à une forme dimère de gp120 et en ce qu'il a un poids moléculaire de l'ordre de 270 kDa.

**4.** Antigène purifié du rétrovirus HIV-2 EHO ou une glycoprotéine ayant les mêmes propriétés immunologiques et obtenue à partir d'un variant du rétrovirus HIV-2 EHO selon la revendication 1, caractérisé en ce qu'il correspond au produit mature du précurseur gp120 et en ce qu'il est la glycoprotéine d'enveloppe extracellulaire gp100 ayant un poids moléculaire de l'ordre de 100 kDa .

5. Antigène purifié ou une glycoprotéine ayant les mêmes propriétés immunologiques et obtenue à partir d'un variant de HIV-2 EHO selon la revendication 1, caractérisé en ce qu'il s'agit d'une glycoprotéine gp36, ayant un poids moléculaire de l'ordre de 36 kDa.

6. Antigène purifié ou une glycoprotéine ayant les mêmes propriétés immunologiques et obtenue à partir d'un variant de HIV-2 EHO selon la revendication 1, caractérisé en ce qu'il s'agit d'une glycoprotéine gp80 et correspond à la forme dimère de gp36 selon la revendication 5.

7. Antigène purifié du rétrovirus HIV-2 EHO ou un produit non glycosylé de la glycoprotéine ayant les mêmes propriétés immunologiques et obtenue à partir d'un variant du rétrovirus HIV-2 EHO, caractérisé en ce qu'il s'agit d'une protéine p60 ayant la même composition en acides aminés que la glycoprotéine gp120 selon la revendication 2 et en ce qu'il ne possède pas de chaînes oligosaccharidiques.

8. Composition pour le diagnostic in vitro d'une infection notamment due à un rétrovirus humain du sous-type HIV-2 EHO, caractérisée en ce qu'elle comprend au moins un antigène selon l'une quelconque des revendications 1 à 7.

9. Composition selon la revendication 8, caractérisée en ce qu'elle contient le précurseur gp120 de la glycoprotéine d'enveloppe, ayant un poids moléculaire de l'ordre de 120 kDa.

10. Composition selon la revendication 8, caractérisée en ce qu'elle contient en outre la glycoprotéine d'enveloppe extracellulaire gp100, ayant un poids moléculaire de l'ordre de 100 kDa.

11. Composition selon la revendication 8, caractérisée en ce qu'elle contient en outre la glycoprotéine dimère gp270, ayant un poids moléculaire de l'ordre de 270 kDa.

12. Anticorps polyclonaux produits chez l'animal, caractérisés en ce qu'ils reconnaissent un antigène purifié selon l'une quelconque des revendications 1 à 7 et en ce qu'ils ne reconnaissent pas les glycoprotéines d'enveloppe de HIV-2 ROD ou les glycoprotéines d'enveloppe de HIV-1.

13. Anticorps monoclonaux excepté un anticorps reconnaissant spécifiquement un épitope localisé dans la séquence d'acides aminés de la glycoprotéine transmembranaire de HIV-2 ROD comprise entre les résidus d'acides aminés en positions 579 et 604, caractérisés en ce qu'ils reconnaissent un épitope qui est commun aux antigènes purifiés selon les revendications 2 à 4 et qui n'est pas reconnu par des anticorps dirigés contre la gp300 ou par des anticorps dirigés contre l'une des glycoprotéines reliées à l'enveloppe gp140 ou gp125 du sous-type de rétrovirus HIV-2 ROD.

14. Anticorps monoclonaux excepté un anticorps reconnaissant spécifiquement un épitope localisé dans la séquence d'acides aminés de la glycoprotéine transmembranaire de HIV-2 ROD comprise entre les résidus d'acides aminés en positions 579 et 604, caractérisés en ce qu'ils reconnaissent spécifiquement la glycoprotéine gp100 selon la revendication 4, ledit épitope n'étant pas reconnu par des anticorps dirigés contre la gp300 ou par des anticorps dirigés contre l'une des glycoprotéines gp140 ou gp125 du sous-type HIV-2 ROD.

15. Anticorps monoclonaux excepté un anticorps reconnaissant spécifiquement un épitope localisé dans la séquence d'acides aminés de la glycoprotéine transmembranaire de HIV-2 ROD comprise entre les résidus d'acides aminés en positions 579 et 604, caractérisés en ce qu'ils reconnaissent spécifiquement la glycoprotéine gp120 selon la revendication 2, ledit épitope n'étant pas reconnu par des anticorps dirigés contre la gp300 ou par d es anticorps dirigés contre l'une des glycoprotéines gp140 ou gp125 du sous-type HIV-2 ROD.

16. Anticorps monoclonaux caractérisés en ce qu'ils reconnaissent spécifiquement la glycoprotéine gp270 selon la revendication 3, ledit épitope n'étant pas reconnu par d es anticorps dirigés contre la gp300 ou par d es anticorps dirigés contre l'une des glycoprotéines gp140 ou gp125 du sous-type HIV-2 ROD.

17. Kit de diagnostic in vitro d'une infection due à un rétrovirus du sous-type HIV-2 EHO dans un échantillon biologique devant être dosé pour la présence d'anticorps contre HIV-2 EHO, caractérisé en ce qu'il comprend:

- un antigène selon l'une quelconque des revendications 1 à 7 ou une composition d'antigènes selon l'une quelconque des revendications 8 à 11, éventuellement marqué,
- des moyens permettant la réaction pour la formation du complexe immunologique formé entre les antigènes précédents et les anticorps si lesdits anticorps sont présents dans l'échantillon biologique à tester, le cas échéant un ou plusieurs tampons d'incubation,
- un témoin négatif,
- des moyens de détection du complexe immunologique formé entre lesdits antigènes et les anticorps si lesdits anticorps sont présents dans l'échantillon.

18. Kit de diagnostic in vitro d'une infection due à un rétrovirus HIV-2 ROD ou HIV-2 EHO dans un échantillon biologique devant être dosé pour la présence d'anticorps contre HIV-2 ROD ou HIV-2 EHO, caractérisé en ce qu'il comprend:

- des antigènes de HIV-2 EHO, selon l'une quelconque des revendications 1 à 7, en combinaison avec les antigènes correspondants de HIV-2 ROD présentant des propriétés immunologiques différentes,
- des moyens permettant la réaction pour la formation du complexe immunologique formé entre les antigènes précédents et les anticorps éventuellement présents dans l'échantillon biologique à tester, le cas échéant un ou plusieurs tampons d'incubation,
- un témoin négatif,
- des moyens de détection du complexe immunologique formé entre lesdits antigènes et les anticorps si lesdits anticorps sont présents dans l'échantillon.

19. Procédé de préparation d'un antigène purifié selon l'une quelconque des revendications 1 à 7, comprenant les étapes suivantes:

- lyse de cellules infectées par un rétrovirus humain HIV-2 EHO et séparation du surnageant de l'extrait cellulaire,
- incubation de l'extrait dilué avec un sérum d'un malade infecté par le rétrovirus HIV-2 EHO ou avec des anticorps polyclonaux ou monoclonaux selon l'une quelconque des revendications 12 à 15 sur une colonne d'immunoaffinité dans des conditions suffisantes pour permettre la formation d'un complexe entre lesdits anticorps et antigènes de l'extrait cellulaire,
- lavage de la colonne d'immunoaffinité pour éliminer les molécules qui ne sont pas retenues sur le support,
- élution des antigènes récupérés par immunoprécipitation dans un tampon d'échantillon pour l'électrophorèse,
- séparation des antigènes élués.

20. Procédé de diagnostic in vitro d'une infection spécifiquement due à un rétrovirus HIV-2 EHO dans un échantillon biologique devant être dosé pour la présence éventuelle d'anticorps contre des antigènes de HIV-2 EHO, comprenant les étapes suivantes:

- mise en contact de l'échantillon biologique à tester avec un antigène selon l'une quelconque des revendications 1 à 7 ou une composition selon l'une quelconque des revendications 8 à 11, dans des conditions permettant la formation d'un conjugué entre lesdits antigènes et les anticorps éventuellement présents dans l'échantillon biologique,
- détection du conjugué immunologique formé, s'il en existe.

21. Procédé de diagnostic in vitro d'une infection dans un échantillon biologique devant être dosé pour la présence éventuelle d'anticorps contre des antigènes de HIV-2 ROD ou HIV-2 EHO, comprenant les étapes suivantes:

- mise en contact de l'échantillon biologique à tester avec un antigène selon l'une quelconque des revendications 1 à 7 ou une composition selon l'une quelconque des revendications 8 à 11, en combinaison avec les antigènes correspondants de HIV-2 ROD, présentant certaines différences immunologiques avec ceux de HIV-2 EHO notamment en ce qui concerne la réaction avec les anticorps contre gp300, dans des conditions permettant la formation d'un conjugué entre lesdits antigènes et les anticorps éventuellement présents dans l'échantillon biologique,
- détection du conjugué immunologique formé, s'il en existe.

Figure 1

a

ROD   EHO

gp300→ ◄    ■ ←gp270

gp140→
gp125→ ◄    .. ←gp120
       ● ←gp100

b

ROD   EHO

gp300→ ●    ■ ←gp270
gp140→ ●    ■ ←gp120
gp80→  ▪    ■ ←gp80

Figure 2

Figure 3

ROD EHO

V$_8$:  −  +     −  +

gp140 →

gp120 →

Figure 4

Figure 5

Figure 6